# FASCICULE DE BREVET EUROPEEN

(11) **EP 0 891 169 B1**
(45) Date de publication et mention de la délivrance du brevet: **28.12.2005**
(21) Numéro de dépôt: 97919466.9
(22) Date de dépôt: 02.04.1997
(51) Int. Cl.: A61F 2/44

(54) **SYSTEME DE CONTENTION ET DE FUSION INTERSOMATIQUE**
STÜTZ- UND FUSIONSSYSTEM FÜR ZWISCHENWIRBELBEREICH
INTERSOMATIC SETTING AND FUSION SYSTEM

(30) Priorité: 03.04.1996 FR 9604196
(43) Date de publication de la demande: 20.01.1999
(73) Titulaire: SCIENT'X S.A.R.L., 75007 Paris (FR)
(72) Inventeur: BENEZECH, Jacques, F-34000 Montpellier (FR); ALBY, Albert, F-75016 Paris (FR)
(74) Mandataire: Thibault, Jean-Marc
(86) Numéro de dépôt international: PCT/FR1997/000591
(87) Numéro de publication internationale: WO 1997/037620

(56) Documents cités:
- EP-A- 0 179 695
- EP-A- 0 298 233
- WO-A-95/08306
- DE-A- 4 302 397
- DE-U- 8 807 485
- FR-A- 2 703 580
- US-A- 4 599 086

## Description

La présente invention concerne un système de contention et de fusion intersomatique des vertèbres comportant au moins une cage interne ouverte recevant de l'os spongieux et destinée à être interposée entre deux vertèbres lors de dissectomie et à pouvoir procéder à la mise en place d'un greffon osseux, ou d'un matériau favorisant la fusion intersomatique qui favorisera la fusion des deux vertèbres concernées.

De telles cages sont connues par exemple, par les demandes de brevet FR2703580, EP493698, EP599419, FR2124815, EP307241, EP615428.

Ces cages sont de forme globalement parallélipipédiques ou ovoïdes. Elles sont rigides, métalliques ou en matériau biocompatible et destinées à recevoir de l'os spongieux grâce à des faces supérieure et inférieure ouvertes.

Il est connu par ailleurs, que le document EP 0 179 695 une prothèse comportant :
- une cage interne avec des faces supérieure et inférieure ouverte pour recevoir un greffon osseux destinée a être interposée entre deux vertèbres lors d'une dissectomie,
- et une plaque externe s'étendant dans un plan sensiblement perpendiculaire au plan d'introduction de la cage, de part et d'autre de celle-ci, et disposant de moyens d'ancrage sur au moins les deux vertèbres adjacantes à solidariser entre elles par l'intermédiaire de la cage.

Il est connu également, que le document FR 2 703 580 une cage de forme parallélépipédique mais qui ne comporte pas de plaque de fixation sur les vertèbres.

Si les cages du type précité donnent généralement de bons résultats, il n'en reste pas moins vrai que dans certains cas il est nécessaire d'assurer le positionnement et la « non-mobilité» de la cage afin de se mettre à l'abri, d'une façon sûre, d'une migration possible ou d'éviter tout risque de déplacement secondaire de la cage.

L'invention vise à atteindre ces objectifs et concerne à cet effet un système de contention et de fusion intersomatique des vertèbres conforme à la revendication 1.

La dite cage est rigide, en métal ou matériau plastique biocompatible, de forme globalement parallélépipédique et adaptée à l'espace intervertébral et prévue pour recevoir de l'os spongieux ou matériau substitut osseux grâce à ses faces supérieure et inférieure ouvertes et/ou par une ouverture frontale.

Le système de l'invention est réalisé selon les besoins sous une forme cage-plaque monobloc ou sous forme d'une cage interne et d'une plaque externe, comportant des moyens d'assemblage de la plaque à la cage.

La présente invention concerne également les caractéristiques qui ressortiront au cours de la description qui va suivre et qui devront être considérées isolément ou selon toutes leurs combinaisons techniques possibles.

Cette description donnée à titre d'exemple non limitatif fera mieux comprendre comment l'invention peut être réalisée, en référence aux dessins annexés, sur lesquels:
- la figure 1 est une vue en perspective éclatée d'un système de contention selon un premier exemple de réalisation.
- la figure 2 est une vue en perspective éclatée d'un système de contention selon un second exemple de réalisation de l'invention, conformément à l'objet de la revendication 1.
- la figure 3 est une vue d'un système de contention monobloc.

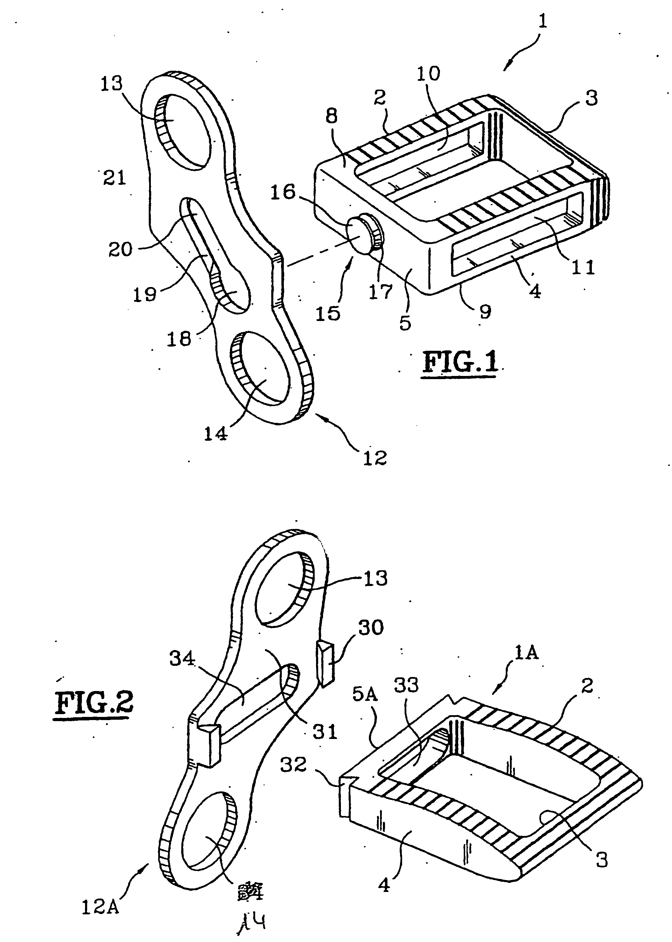

A titre d'exemple non limitatif, la cage 1 représentée sur la figure 1 est constituée globalement par un parallélépipède rigide dont les parois latérales 2, 3, 4, 5 sont destinées à emprisonner de l'os spongieux ou substitut osseux et dont les faces supérieure 8 et inférieure 9 sont ouvertes vers deux vertèbres successives.

Les faces antérieure 5 et postérieure 3 ont de hauteurs déterminées afin d'assurer la conservation d'un espace intervertébral approprié.

Toujours selon le présent exemple, les parois latérales 2 et 4 de la cage 1 sont pourvues de larges lumières 10 et 11 de forme semblable à celles des parois latérales correspondantes 2 et 4 dans lesquelles elles sont percées.

La cage 1 est ainsi pourvue d'ouvertures sur quatre de ses faces. La cage 1 comporte par ailleurs sur sa face antérieure 5 un élément externe formant une bride 12 (désignée par la suite par plaque) s'étendant dans un plan sensiblement perpendiculaire au plan d'introduction de la cage 1, de part et d'autre de celle-ci, et disposant à chacune de ses extrémités de moyens d'ancrage 13 et 14 sur au moins deux vertèbres adjacentes à solidariser entre elles par l'intermédiaire de la cage 1.

Bien entendu, la bride externe ou plaque 12 pourrait être obtenue de manière monobloc avec la cage interne 1, par exemple par moulage de l'ensemble cage-plaqué.

Néanmoins, il a été préféré selon le présent exemple de réalisation, de présenter une plaque 12 et une cage interne 1 réalisées distinctement et solidarisées entre elles ultérieurement par l'intermédiaire de moyens d'assemblage.

Toujours selon l'exemple de la figure 1, ces moyens d'assemblage de la plaque 12 sur la cage 1 sont constitués par un pion 15 cylindrique réalisé sur une face antérieure verticale 5 de la dite cage et une gorge périphérique 17 aménagée sous la tête circulaire 16 du dit pion 15, la dite tête 16 étant susceptible d'être introduite dans une partie correspondante d'une zone élargie 18 d'une lumière oblongue. 19 en forme de boutonnière percée dans la plaque 12, et la dite gorge 17 étant de dimension telle à pouvoir coulisser entre les bords latéraux d'une zone rétrécie 20 de ladite boutonnière jusqu'à ce que la tête 16 du pion 15 coopère en verrouillage axial avec la face externe 21 de la plaque 12.

On peut prévoir qu'un tel engagement du pion dans la boutonnière puisse se faire à force dans la zone rétrécie.

Bien entendu, la cage qui vient d'être décrite ci-dessus pourra avoir des dimensions différentes tant en hauteur qu'en largeur et en profondeur.

Elle pourra également avoir comme dans l'exemple qui va suivre, une forme anatomique préférée.

On peut également supposer que la pose de l'os spongieux ou autre substitut osseux sera faite préalablement ou après positionnement de la cage 1 entre les vertèbres.

L'exemple de réalisation représenté sur la figure 2 diffère essentiellement du précédent en ce que les moyens d'assemblage de la plaque 12A sur la cage 1A sont constitués par une glissière en queue d'aronde dont la partie formant mortaise 30 est réalisée sur une face interne 31 de la plaque 12A dans un sens de coulissement vertical et la partie tenon correspondante 32 sur la face frontale externe 5A de la cage 1A, ou inversement.

On notera que selon le présent exemple de réalisation, la cage 1A ne comporte plus d'ouvertures latérales comme dans l'exemple précédent.

En fait, la face frontale externe 5A de la cage 1A et la face correspondante 31 de la plaque 12A avec laquelle elle coopère comportent chacune des lumières oblongues identiques 33, 34 destinées à être mises en .coïncidence lors de l'assemblage de la plaque 12A sur la cage 1A, de manière à permettre l'introduction de l'os spongieux, frontalement après mise en place de la cage 1A.

On remarquera également la forme et le profil particulier de la cage 1A selon l'exemple de la figure 2 permettant une adaptation parfaite à l'espace intervertébral.

L'exemple de réalisation représenté sur la figure diffère de l'exemple de la figure 2 en ce qu'il est réalisé de manière monobloc.

La face antérieure 5A de la cage-plaque comporte un lumière oblongue 33 de manière à permettre d'introduire frontalement de l'os spongieux dans la partie cage après mise en place de la dite cage-plaque.

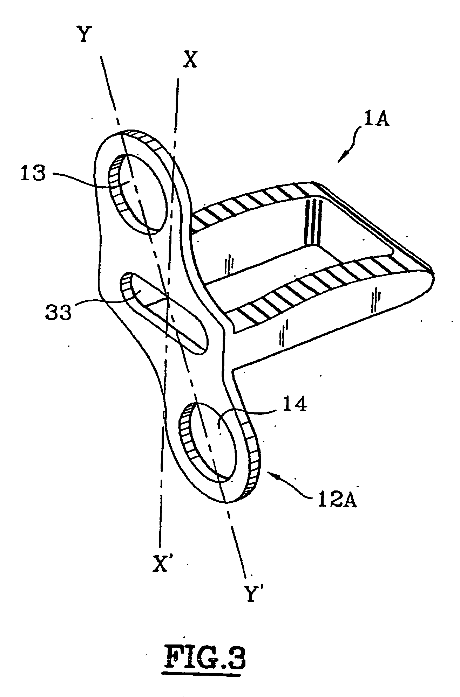

D'une manière commune aux exemples de réalisation de figures 1, 2 et 3 qui viennent d'être citées, les moyens d'ancrage de la plaque 12, 12A sur les vertèbres, après fixation de la cage 1, 1A, s'effectuent par l'intermédiaire de vis pédiculaires (non représentées) traversant des trous correspondants 13, 14 pratiqués aux extrémités de ladite bride 12, 12A.

IL est à noter, selon une autre caractéristique de l'invention, que les trous de fixation 13, 14 de la plaque 12, 12A sont disposés de part et d'autre d'un axe vertical médian X, X' de l'ensemble, sur une diagonale Y, Y'.

Selon un mode de réalisation non représenté, le système de contention peut mettre en oeuvre deux ou plus cages internes 1, 1A solidarisées entre elles et par rapport à trois ou plus vertèbres successives par l'intermédiaire d'une plaque 12, 12A comportant deux moyens d'assemblage 15, 18 ou 30, 32 avec lesdites cages 1, 1A et deux trous de fixation d'extrémité 13, 14 et un trou intermédiaire coopérant avec trois ou plus vis pédiculaires d'ancrage sur les vertèbres.

Les plaques sont représentées avec une vis de fixation par vertèbre mais elles peuvent en comporter davantage, 2 par exemple.

Il est bien entendu que le système de contention peut également être constitué de deux ou plusieurs ensembles cage-plaque tels que définis dans les exemples des figures 1, 2 ou 3 reliés entre eux par les dites plaques se chevauchant on non, deux par deux.

Les systèmes de contention de l'invention sont réalisés en alliage de titane ou matériau équivalent ou bien en matière plastique bio-compatible.

## Revendications

1. Système de contention et de fusion intersomatique des vertèbres du type comportant :
- au moins une cage interne (**1A**) avec des faces supérieure (**8**) et inférieure (**9**), ouverte pour recevoir de l'os spongieux ou substitut osseux et destinée à être interposée entre deux vertèbres lors d'une dissectomie,
- et une plaque externe (**12A**) s'étendant dans un plan sensiblement perpendiculaire au plan d'introduction de la cage (**1A**), de part et d'autre de celle-ci, et disposant de moyens d'ancrage (**13, 14**) sur au moins les deux vertèbres adjacentes à solidariser entre elles par l'intermédiaire de la cage (**1A**),
**caractérisé en ce que** la cage **(1A)** est composée d'une paroi antérieure (**5A**), d'une paroi postérieure (**3**) et de deux parois latérales (**2, 4**), ces parois formant un parallélépipède rigide avec la face supérieure (**8**) convexe en vue latérale, de sorte que la forme et le profil de la cage sont adaptés à l'espace, intervertébral délimité par deux vertèbres adjacentes, et **en ce que** la cage (**1A**) est équipée d'une plaque externe (**12A**) présentant, à chacune de ses extrémités, en tant que moyens d'ancrage, un seul trou de fixation pour le passage d'une vis d'ancrage dans la vertèbre adjacente, lesdits trous (**13, 14**) étant disposés sur une diagonale (**y-y'**) de part et d'autre d'un axe vertical médian (**x-x'**) de l'ensemble.

2. Système selon la revendication 1, **caractérisé en ce que** la plaque externe (**12A**) et la paroi antérieure (**5A**) la cage **(1A)** comportent chacune une lumière oblongue (**34, 33**) permettant d'introduire frontalement de l'os spongieux dans la cage.

3. Système selon la revendication 1; **caractérisé en ce que** la plaque externe (**12A**) est obtenue de manière monobloc avec la cage interne (**1A**).

4. Système selon la revendication 1, **caractérisé en ce que** la plaque externe (**12**) et la cage interne (**1A**) sont réalisées distinctivement et solidarisées entre elles ultérieurement par l'intermédiaire de moyens d'assemblage.

5. Système selon la revendication 4, **caractérisé en ce que** les moyens d'assemblage de la plaque (**12A**) sur la cage (**1A**) sont constitués par une glissière en queue d'aronde dont la partie formant mortaise (**30**) est réalisée sur une face interne (**31**) de la plaque (**12A**) dans un sens de coulissement vertical et la partie tenon correspondante (**32**) sur la face frontale externe (**5A**) de la cage (**1A**), ou inversement.

6. Système selon la revendication 5, **caractérisé en ce que** la face frontale externe (**5A**) de la cage (**1A**) et la face correspondante (**31**) de la plaque (**12A**) avec laquelle elle coopère comportent chacune des lumières oblongues identiques (**33, 34**) destinées à être mises en coïncidence lors de l'assemblage de la plaque (**12A**) sur la cage (**1A**), de manière à permettre l'introduction de l'os spongieux, frontalement après mise en place de la cage (**1A**).

7. Système selon la revendication 1, **caractérisé en ce que** la cage interne comporte, outre des faces, supérieure (**8**) et inférieure (**9**) ouvertes, des parois latérales (**2, 4**) pourvues de lumières d'introduction (**10,11**) de l'os spongieux.

8. Système selon l'une des revendications précédentes, **caractérisé en ce qu'**il met en oeuvre au moins deux cages internes (**1A**) solidarisées entre elles et par rapport à au moins trois vertèbres successives par l'intermédiaire d'une plaque (12A) comportant deux moyens d'assemblage (**30, 32**) avec lesdites cages (**1A**) et deux trous de fixation d'extrémité (**13, 14**) et un trou de fixation intermediare coopérant avec au moins trois vis d'ancrage sur les vertèbres.

## Patentansprüche

1. System zur intersomatischen Kontention und Fusion von Wirbeln, umfassend:
- mindestens ein Innengehäuse (1A) mit einer oberen Fläche (8) und einer unteren Fläche (9), das offen ist, um einen Spongiaknochen oder ein knöchernes Substitut aufzunehmen und das dazu bestimmt ist, im Falle einer Dissektomie zwischen zwei Wirbeln angeordnet zu werden,
- und eine äußere Platte (12A), die sich in einer im wesentlichen zur Einführungsebene des Gehäuses (1A) senkrechten Ebene auf beiden Seiten des Gehäuses erstreckt und die über Mittel (13, 14) zur Verankerung auf mindestens den zwei benachbarten Wirbeln verfügt, die miteinander mit Hilfe des Gehäuses (1A) fest verbunden werden sollen,
**dadurch gekennzeichnet,**
- **daß** das Gehäuse (1A) aus einer Vorderwand (5A), einer Rückwand (3) und zwei Seitenwänden (2, 4) besteht, wobei diese Wände ein festes Parallelepiped mit der oberen Fläche (8) bilden, das von der Seite gesehen konvex ist, so daß die Form und das Profil des Gehäuses an den Zwischenwirbelraum angepaßt sind, welcher durch zwei benachbarte Wirbel begrenzt wird, und
- **daß** das Gehäuse (1A) mit einer äußeren Platte (12A) versehen ist, welche auf jedem ihrer Enden, die als Mittel zur Verankerung dienen, eine einzige Befestigungsöffnung für das Einführen einer Verankerungsschraube in den benachbarten Wirbel aufweist, wobei die Öffnungen (13, 14) auf einer Diagonale (y-y') auf beiden Seiten einer vertikalen medianen Achse (x-x') des Zusammenbaus angeordnet sind.

2. System nach Anspruch 1, **dadurch gekennzeichnet, daß** die äußere Platte (12A) und die Vorderwand (5A) des Gehäuses (1A) jeweils einen länglichen Schlitz (34, 33) aufweisen, welcher es ermöglicht, Spongiaknochen frontal in das Gehäuse einzuführen.

3. System nach Anspruch 1, **dadurch gekennzeichnet, daß** die äußere Platte (12A) in einem Stück mit dem Innengehäuse (1A) erhalten wird.

4. System nach Anspruch 1, **dadurch gekennzeichnet, daß** die äußere Platte (12) und das Innengehäuse (1A) getrennt hergestellt und zu einem späteren Zeitpunkt miteinander mit Hilfe von Mitteln zum Verbinden zu einer Einheit verbunden werden.

5. System nach Anspruch 4, **dadurch gekennzeichnet, daß** die Mittel zum Verbinden der Platte (12A) mit dem Gehäuse (1A) durch eine Führung in Schwalbenschwanzform gebildet werden, deren eine Nut (30) bildender Abschnitt auf einer Innenfläche (31) der Platte (12A) in einer vertikalen Gleitrichtung und deren entsprechender Zapfenabschnitt (32) auf der äußeren Vorderwand (5A) des Gehäuses (1A) ausgebildet ist, oder umgekehrt.

6. System nach Anspruch 5, **dadurch gekennzeichnet, daß** die äußere Vorderwand (5A) des Gehäuses (1A) und die entsprechende Fläche (31) der Platte (12A), mit welcher sie zusammenwirkt, jeweils identische längliche Schlitze (33, 34) aufweisen, die dazu bestimmt sind, zum Zeitpunkt des Anbringens der Platte (12A) auf dem Gehäuse (1A) derart zusammengeführt zu werden, daß das frontale Einführen des Spongiaknochens nach der Positionierung des Gehäuses (1A) ermöglicht wird.

7. System nach Anspruch 1, **dadurch gekennzeichnet, daß** das Innengehäuse zusätzlich zu der oberen Fläche (8) und der unteren Fläche (9), die offen sind, Seitenwände (2, 4) umfaßt, die mit Schlitzen (10, 11) zum Einführen des Spongiaknochens versehen sind.

8. System nach einem der vorhergehenden Ansprüche, **dadurch gekennzeichnet, daß** es mindestens zwei Innengehäuse **(1A)** verwendet, welche untereinander und in Bezug auf mindestens drei aufeinanderfolgende Wirbel mit Hilfe einer Platte **(12A)** fest verbunden sind, welche zwei Mitteln **(30, 32)** zum Verbinden mit den Gehäusen **(1A)** und zwei Befestigungsöffnungen **(13, 14)** an den Enden und eine Zwischen-Befestigungsöffnung umfaßt, welche mit mindestens drei Verankerungsschrauben auf den Wirbeln zusammenwirken.

## Claims

1. An intersomatic fusion and setting system for vertebrae of the type comprising:
- at least an internal cage (1A) with open top and bottom faces (8, 9) for receiving spongy bone or bone substitute and designed to be interposed between two vertebrae during diskectomy; and
- an external plate (12A) extending in a plane substantially perpendicular to the insertion plane of the cage (1A), on either side thereof, and having anchor means (13, 14) for anchoring at least to the two adjacent vertebrae to be secured together by means of the cage (1A),
the system being **characterised in that** the cage (1A) has an anterior wall (5A), a posterior wall (3), and two side walls (2, 4), said walls forming a rigid parallelepiped with the top convex face (8) in cross-section, such that the shape and profile of the cage are adapted to the intervertebral space defined by two adjacent vertebrae, and **in that** the cage (1A) is fitted with an external plate (12A) having, at each end, anchor means constituted by respective single fixing holes for passing respective screws for anchoring in the adjacent vertebra, said holes (13, 14) being disposed on a diagonal (y-y') on either side of a central vertical axis (x-x') of the assembly.

2. A system according to claim 1, **characterised in that** the external plate (12A) and the anterior wall (5A) of the cage (1A) have respective oblong slots (33, 34) enabling spongy bones to be inserted frontally into the cage.

3. A system according to claim 1, **characterised in that** the external plate (12A) is obtained as a single piece with the internal cage (1A).

4. A system according to claim 1, **characterised in that** the external plate (12) and the internal cage (1A) are made separately and are secured to each other subsequently by assembly means.

5. A system according to claim 4, **characterised in that** the means for assembling the plate (12A) on the cage (1A) are constituted by a dovetail slideway whose mortice-forming portion (30) is formed on an inside face (31) of the plate (12A) in a vertical sliding direction and whose corresponding tenon portion (32) is formed on the outside front face (5A) of the cage (1A), or vice versa.

6. A system according to claim 5, **characterised in that** the outside front face (5A) of the cage (1A) and the corresponding face (31) of the plate (12A) with which it co-operates have respective identical oblong slots (33, 34) designed to be put into coincidence when the plate (12A) is assembled to the cage (1A), so as to enable spongy bone to be inserted frontally after the cage (1A) has been put into place.

7. A system according to claim 1, **characterised in that** in addition to open top and bottom faces (8, 9), the internal cage has side walls (2, 4) provided with insertion slots (10, 11) for spongy bone.

8. A system according to one of the preceding claims, **characterised in that** it implements at least two internal cages (1A) secured to one another and relative to at least three successive vertebrae by means of a plate (12A) having two assembly means (30, 32) for being assembled to said cages (1A), and two end fixing holes (13, 14), and one intermediate fixing hole co-operating with at least three screws for anchoring in the vertebrae.
